# EUROPÄISCHE PATENTANMELDUNG

(11) **EP 4 223 152 A1**
(43) Veröffentlichungstag der Anmeldung: **09.08.2023**
(21) Anmeldenummer: 23000014.3
(22) Anmeldetag: 30.01.2023
(51) Int. Cl.: A24B 15/16, A24B 15/30, A61K 31/352

(54) **TABAKFREIE RAUCHMISCHUNG FÜR WASSERPFEIFEN (SHISHAS) ODER NORMALE PFEIFEN AUF BASIS VON CANNABIDIOL-HANF (CBD-HANF) ZUM SOFORTIGEN KONSUM**

(30) Priorität: 03.02.2022 DE 202022000286 U
(71) Anmelder: Hopfe, Andreas, 06110 Halle (DE)
(72) Erfinder: Hopfe, Andreas, 06110 Halle (DE)
(74) Vertreter: Braeter, Alexander

(57) **Zusammenfassung**

Bezeichnung

• tabakfreie Rauchmischung (RM) für Wasserpfeifen (Shishas) zur Verwendung in einem Shisha-Kopf oder in normalen Pfeifen auf Basis von Cannabidiol-Hanf (CBD-Hanf) zum sofortigen Konsum.

Kurzfassung

• Die Erfindung betrifft ein alternatives Trägermaterial (TM).
• Die Erfindung betrifft ferner ein Kit einer konsumfertigen Rauchmischung (RM) enthaltend ein vorbefeuchtetes Trägermaterial (TM).
• Beschrieben wird ferner ein Verfahren zum Befeuchten und Aromatisieren des alternativen Trägermaterials.
• Beschrieben wird außerdem ein Verfahren zur Verhinderung des Kontaktes von Asche- und Kohlepartikeln beim Dampfen in einer Shishapfeife durch Verwendung eines Silikon- bzw. Glaskopfs für Wasserpfeifen (Shishas) mitsamt eines Platten- oder Kaminaufsatzes.

## Beschreibung

### Gebiet der Erfindung

Die Erfindung betrifft eine tabakfreie Rauchmischung für Wasserpfeifen (Shishas) zur Verwendung in einem Shisha-Kopf auf Basis von Cannabidiol-Hanf (CBD-Hanf) zum sofortigen Konsum, die sowohl geraucht als auch verdampft werden kann. Die Unterschiede von Dampfen und Rauchen in der Shisha werden weiter unten erläutert.

### Hintergrund der Erfindung und Stand der Technik

Das Shisha-Rauchen ist seit Jahrhunderten Teil der asiatischen und nahöstlichen Kultur und ist ein gesellschaftlicher Brauch, der vor allem in Gruppen gepflegt wird. Shisha-Rauchen ist eine beliebte Methode des Tabakrauchens, bei der der Rauch durch ein Wasserpfeife (Shisha) gezogen wird. In vielen Städten gibt es Cafés und Bars, in denen entsprechende Shishapfeifen und der dazugehörige Tabak angeboten wird.

In der Regel wird ein mit Molasse aromatisierter Shisha-Tabak (oder das unten beschriebene Trägermaterial) auf einen Einlass der Wasserpfeife gelegt, der mit brennender Kohle (heute zumeist aus gepressten Kokosfasern bestehend) in Kontakt kommt, um den Rauch zu erzeugen, der schließlich vom Benutzer inhaliert wird. Sobald der Rauch erzeugt ist, wandert der Rauch der Shisha durch den Shisha-Kopf in einen Wasserbehälter, wo er abgekühlt wird, bevor er durch die Pfeife über einen Schlauch und das am Ende befindliche Mundstück zum Mund des Benutzers gezogen wird. Traditionell wird der Shisha-Tabak mit einer Pinzette/Zange in den Shisha-Kopf gefüllt, welcher auf das obere Ende der Rauchsäule gesteckt wird.

Für die traditionelle Shisha wird als Trägermaterial normalerweise ein spezieller sehr "feuchter" Tabak verwandt, der in den Ländern, in denen ein hoher Feuchtigkeitsgehalt zulässig ist, einen Feuchtigkeitsgehalt von 25 bis 40 Gewichtsprozent hat.

### Unterschied von Dampfen und Rauchen in der Shisha

Entstehen beim Erhitzen Rauchpartikel, flüchtige Kohlenwasserstoffe oder sogar durch Verbrennung herunterfallender Asche polyzyklische Kohlenstoffe dann spricht man vom Shisha rauchen. Bei der Verwendung von Tabak ist das Entstehen von Rauch praktisch nicht zu vermeiden. Besteht der Nebel lediglich aus Flüssigkeitströpfchen dann spricht man vom Shisha dampfen. Das Verdampfen ähnelt insoweit der Anwendung im Vaporisator, um die Wirkstoffe und Aromen aus dem aromatisierten Shisha-Tabak (oder dem unten beschriebene Trägermaterial) zu gewinnen, um diese zu inhalieren.

Die Voraussetzung für einen sicheren Konsum des Dampfproduktes ist, dass unter keinen Umständen Holzkohlenpartikel oder Holzkohlenasche, insbesondere keine glühenden Bestandteile, auf den Trägerstoff fallen. Ferner ist unbedingt darauf zu achten, dass der Träger nicht überhitzt wird. Es ist daher unbedingt sicherzustellen, dass der Träger weder unmittelbaren Kontakt zur Glut hat, noch Kontakt zu Metallteilen, die von der glühenden Kohle erhitzt werden (z.B. das Sieb des Kohleträgers). Ansonsten ist kein Dampfen möglich.

Üblicherweise wird ein Standard Shisha-Kopf mit Kaminaufsatz verwendet. Diese Teile sind so gebaut, dass Sie Öffnungen nach unten haben und man den Tabak durch die Öffnungen sehen kann. Wird nun die glühende Kohle auf den Träger gelegt, natürlich verbrannt und auch noch hin und her bewegt wird, fällt unvermeidlich heiße Asche durch die Löcher. Damit fällt immer Asche oder glühende Kohleteilchen auf den Trägerstoff, wenn keine Vorkehrungen getroffen werden. Um dies zu vermeiden, kann statt eines Standard Shisha-Kopfes z.B. ein Silikon- bzw. Glaskopf mit einem Platten- oder Kaminaufsatz verwendet werden.

### Traditioneller Shisha-Tabaks

Der für Wasserpfeifen normalerweise als Trägermaterial verwendete Tabak wird je nach Benutzer mit Zuckermelasse und anderen Zutaten gemischt. Als Befeuchtungsmittel dient vor allem Glycerin. Dazu werden üblicherweise je nach Rezeptur Honig oder vielerlei Arten von Zucker und Aromen beigemischt. Es gibt hunderte von Aromen, die beigemischt werden können. Die Aromastoffe sind üblicherweise in Propylenglykol gelöst. Die "Feuchtigkeit" des Shisha-Tabaks beruht auf seinem Gehalt von Glycerin.

Bei der Mischung von Glycerin, Honig, verschiedenen Zuckerarten unter Hinzusetzung von Aroma, spricht man von der Molasse. Da jeder Hersteller seine eigenen Rezepturen als auch unterschiedlichen Qualitäten der Tabaksorten, sprich Verhältnis Blätter/Rippen verwendet, sind auch die Rezepturen und Mischungsverhältnisse unterschiedlich.

Zum Einziehen der Molasse in den Tabak, wird dieser Tabak mehrere Wochen in Behältern oder Fässern aufbewahrt, die regelmäßig bewegt werden müssen.

in einigen Ländern ist bei der Verwendung von Tabak nur ein weitaus geringerer Feuchtigkeitsgehalt in Gewichtsprozent zulässig, sodass Tabak mit einem höheren Feuchtigkeitsgehalt nicht eingeführt oder vertrieben werden darf. Zudem enthält Tabak neben Nikotin, noch Tabakaromen oder andere Tabakbestandteile, die als gesundheitsgefährdend gelten können.

Diese Probleme werden durch die vorliegende Erfindung überwunden.

### Grundzüge der Erfindung

Die Erfindung erlaubt die Herstellung einer tabakfreien Rauchmischung **(RM)** für Wasserpfeifen (Shishas) zur Verwendung in einem Shisha-Kopf oder normale Pfeifen zum sofortigen Konsum.

Beim Herstellungsprozess werden die nachfolgenden Zutaten vollständig oder auch nur Teile davon vermischt.

Als Trägermaterial **(TM)** wird der üblicherweise verwendete Tabak durch die Blüten und Blätter von Cannabidiol-Hanf **(CBD-Hanf)** ersetzt unter Berücksichtigung der länderspezifischen gesetzlichen Rahmenbedingungen.

Für die Herstellung der Molasse **(MO)** werden Glycerin (E422), Zuckermelasse (Zuckersirup als Nebenerzeugnis in der Zuckerproduktion aus Zuckerrohr, Zuckerrüben), Süßungsmittel (Honig, Glucose, Fructose) und Aromastoffe auf Propylenglykol-Basis verwendet.

Die Rauchmischung **(RM)** entsteht, indem das Trägermaterial **(TM)** mit der Molasse **(MO)** als Befeuchtungsmittel versetzt wird. Damit die Molasse (**MO)** in das Trägermaterial **(TM)** einzieht, wird die Rauchmischung **(RM)** über einen längeren Zeitraum in Behältern oder Fäsern unter regelmäßiger Bewegung aufbewahrt. Die Entnahme der konsumbereiten Rauchmischung **(RM)** als Endprodukt erfolgt üblicherweise nach Ablauf von 4 bis 12 Wochen.

Der Vorteil des Verfahrens ist die sofortige Zurverfügungstellung einer konsumfertigen, tabakfreien Rauchmischung **(RM)** für den Konsumenten oder Betreiber einer entsprechenden Shishabar. Die Rauchmischung **(RM)** kann dann in normalen Pfeifen oder einem Standard Shisha-Kopf mit Kaminaufsatz geraucht oder aber z.B. in einem Silikon- bzw. Glaskopf mit einem Platten- oder Kaminaufsatz verdampft werden. Die in der Molasse **(MO)** enthaltenen Aromen und Süßungsmittel verbessern dabei den Geschmack. Optional können im Rahmen der gesetzlichen Bestimmungen unmittelbar vor dem Rauchen oder Dampfen weitere Stoffe zugegeben werden, z.B. Nikotinshots und/oder Cannabinoide, insbesondere weiterem CBD.

### Beschreibung des Trägermaterials (TM)

Als Trägermaterial werden die Blüten und Blätter des Cannabidiol-Hanfs **(CBD-Hanf)** (getrocknet, prozessbedingt fermentiert, chemisch unbelastet) in zerkleinerter Form verwendet. Dazu wird das Material bevorzugt kleingeschnitten (Schnittlänge kleiner als 6 cm, Gewebeflächen kleiner als 3 cm), damit es einfacher für die Shisha portioniert werden kann.

### Beschreibung der Molasse (MO)

Die Molasse enthält 65 bis 80 % Glycerin, 10 bis 25 % Süßungsmittel (Honig, Glucose, Fructose) und 1 % bis 10 % sonstige Aromastoffe.

Die Aromastoffe sind kommerziell erhältlich, bevorzugt werden sie als Lösung in Propylenglykol verwendet.

Die Rauchmischung kann optional auch Nikotin und/oder Tabakaromen enthalten.

Optional kann die konsumbereite Rauchmischung **(RM)** noch mit Zusatzstoffen angereichert werden, wie z.B. Vitaminen. Auch ein Zusatz von Cannabinoiden, insbesondere weiterem CBD ist im Rahmen der gesetzlichen Bestimmungen möglich.

In der konsumbereiten Rauchmischung **(RM)**, sind typischerweise 1 bis 8 % Aromastoffe enthalten.

Bei der hierin beschriebenen erfindungsgemäßen Herstellung werden pro Portion genau die Menge Aromen abgewogen und abgefüllt, die erwünscht oder festgelegt sind. Man hat somit immer die gleiche Geschmacksqualität für jede Einheit.

### Abgrenzung zum Stand der Technik

Im Internet kursieren Anleitungen zum Befeuchten von fertigem Shishatabak mit Melassen und/oder Wasser. Im Unterschied zur vorliegenden Erfindung handelt es sich dabei um eine (Nachbefeuchtung) von älterem ausgetrocknetem Tabak, der ursprünglich bereits befeuchtet war. Abgesehen von der deutlichen schlechteren Tabakqualität, die durch eine solche "Nachbefeuchtung" erhalten wird, fallen diese Prozeduren nicht in den Bereich der hier beschriebenen Erfindung.

### Verwendung eines alternativen Trägermaterials

Die Erfindung besteht in der Verwendung eines alternativen Trägermaterials (TM), welcher nicht aus Tabak hergestellt wird. Hiermit kann eine tabakfreie Rauchmischung zum Rauchen oder Dampfen hergestellt werden.

Das Trägermaterial wird im gleichen Shishakopf gedampft wie Tabak geraucht wird. Allerdings sind obig beschriebene Anweisungen einzuhalten.

In dieser Ausführungsform können auch Shishas mit elektrischer Heizung Verwendung finden.

### Bezugszeichenliste

- (TM): - Trägermaterial
- (MO): - Molasse
- (RM): - Rauchmischung
- (CBD-Hanf): - Cannabidiol-Hanf

## Patentansprüche

1. Rauchmischung zur Verwendung in Wasserpfeifen (Shishas) oder normalen Pfeifen, enthaltend
**a)** Blüten des CBD-Hanfes, als Trägermaterial
**b)** Molasse als Befeuchtungsmittel, enthaltend 65 bis 95 % Glycerin, 10 bis 20 % Zuckermelasse, 3 bis 7 % Süßungsmittel (Honig, Glucose, Fructose) und sonstige Aromastoffe.

2. Rauchmischung zur Verwendung in Wasserpfeifen (Shishas) oder normalen Pfeifen gemäß Anspruch 4, enthaltend
**a)** 65 bis 75 % Blüten des CBD-Hanfes, als Trägermaterial
**b)** 25 bis 35 % Molasse als Befeuchtungsmittel, enthaltend 65 bis 95 % Glycerin, 10 bis 20 % Zuckermelasse, 3 bis 7 % Süßungsmittel (Honig, Glucose, Fructose) und sonstige Aromastoffe.

3. Rauchmischung zur Verwendung in Wasserpfeifen (Shishas) oder normalen Pfeifen gemäß Anspruch 1 oder 2, zusätzlich enthaltend Aromastoffe, Vitamine, CBD, Tabakaromen und/ oder Nikotin.

4. Rauchmischung für Wasserpfeifen (Shishas) zur Verwendung in einem Shisha-Kopf, enthaltend
**a)** Blüten und Blätter des CBD-Hanfes, als Trägermaterial
**b)** Molasse als Befeuchtungsmittel, enthaltend 65 bis 80 % Glycerin, 10 bis 20 % Süßungsmittel (Honig, Glucose, Fructose) und 1 % bis 10 % sonstige Aromastoffe.

5. Rauchmischung für Wasserpfeifen (Shishas) zur Verwendung in einem Shisha-Kopf gemäß Anspruch 4, enthaltend
**a)** 20 bis 40 % Blüten des CBD-Hanfes, als Trägermaterial
**b)** 60 bis 80 % Molasse als Befeuchtungsmittel, enthaltend 65 bis 80 % Glycerin, 10 bis 20 % Süßungsmittel (Honig, Glucose, Fructose) und 1 % bis 10 % sonstige Aromastoffe.

6. Rauchmischung für Wasserpfeifen (Shishas) zur Verwendung in einem Shisha-Kopf gemäß Anspruch 4 oder 5, zusätzlich enthaltend Aromastoffe, Vitamine, CBD, Tabakaromen und/oder Nikotin.

7. Kit zur Verwendung in Wasserpfeifen (Shishas) zur Verwendung in einem Shisha-Kopf oder in normalen Pfeifen, enthaltend ein oder mehrere Portionen der Rauchmischung gemäß Anspruch 1-6 zum sofortigen Konsum.
